# EUROPEAN PATENT APPLICATION

(11) **EP 4 790 128 A1**
(43) Date of publication of application: **12.08.2026**
(21) Application number: 26154617.0
(22) Date of filing: 28.01.2026
(51) Int. Cl.: C12M 1/00, B01L 3/02, B01L 3/00, C12M 1/26, C12M 1/34, C12M 1/36, G01N 35/10, C12M 1/32

(54) **CELL HANDLING APPARATUS**

(30) Priority: 10.02.2025 JP 2025020233
(71) Applicant: Yamaha Hatsudoki Kabushiki Kaisha, Iwata-shi, Shizuoka 438-8501 (JP)
(72) Inventor: ITO, Saburo, Iwata-shi, Shizuoka, 4388501 (JP)
(74) Representative: Müller-Boré & Partner Patentanwälte PartG mbB

(57) **Abstract**

A cell handling apparatus includes: a container; a camera; a head; and a correction control unit. The container includes a translucent and shape-deformable bottom surface that holds a cell, and a state checking portion for recognizing the shape deformation. A tip is attached to the head and the head executes an operation of approaching the bottom surface from above to suck a cell on the bottom surface and an operation of discharging a cell toward the bottom surface. The correction control unit is configured to correct position coordinates of the head. The correction control unit is configured to detect contact of a tip end of the tip with the bottom surface based on a state change of the state checking portion in an image of the bottom surface captured by the camera.

## Description

### FIELD OF INVENTION

The present invention relates to a cell handling apparatus capable of executing a cell suction or discharge operation on a container containing a cell.

### BACKGROUND ART

For example, in the fields of medicine and biological research, an operation of transferring a cell from a culture container of the cell to a processing container for performing inspection, observation, and the like may be performed. For this operation, a cell handling apparatus that performs an operation of sucking and holding a target cell from the culture container by using a head to which a suction tip is attached, and discharging the held cell to the processing container is used (see, for example, WO 2022/145086 A).

When a cell is sucked, it is necessary to accurately position a target cell and a tip opening of the suction tip. For example, in a case where the tip opening does not reach a height position suitable for sucking a target cell, the target cell cannot be picked. Further, if the tip opening is excessively pressed against a cell culture surface, the tip is damaged. The same problem occurs when a cell is discharged toward a predetermined cell containing portion.

### SUMMARY OF THE INVENTION

An object of the present invention is to provide a cell handling apparatus capable of accurately sucking and discharging a cell.

A cell handling apparatus according to an aspect of the present invention includes: a container; a camera; a head; and a correction control unit. The container includes a translucent bottom surface that holds a cell, and the bottom surface is deformable in shape and has a state checking portion for recognizing the shape deformation. The camera images the bottom surface of the container from below. A tip having a tip end for sucking or discharging a cell is attached to the head, and the head executes an operation of approaching the bottom surface from above to suck a cell on the bottom surface and an operation of discharging a cell toward the bottom surface. The correction control unit is configured to correct position coordinates of the head. The correction control unit is configured to acquire a correction value of the position coordinates of the head by obtaining a difference between a lowering target position of the tip end of the tip and an actual lowering position where the tip end of the tip actually comes into contact with the bottom surface, and detect contact of the tip end of the tip with the bottom surface based on a state change of the state checking portion in an image of the bottom surface captured by the camera.

### BRIEF DESCRIPTION OF THE DRAWINGS

Fig. 1 is a diagram schematically illustrating a configuration example of a cell handling apparatus according to an embodiment of the present invention;
Fig. 2A is a perspective view of a well plate which is an example of a container, Fig. 2B is a plan view of one well, and Fig. 2C is a cross-sectional view of a grid;
Fig. 3A is a cross-sectional view of a tip attached to a head and a diagram illustrating a moving mechanism and a suction mechanism of the tip, Fig. 3B is a cross-sectional view of the tip during a suction operation, and Fig. 3C is an enlarged view of a main part of Fig. 3B;
Fig. 4 is a block diagram illustrating an electrical configuration of a cell transfer device;
Fig. 5 is a diagram illustrating an example of a movement correction operation of the head;
Figs. 6A, 6B, and 6C are schematic diagrams illustrating an example of acquiring a movement correction value of the head;
Fig. 7 is a diagram illustrating an example of an acquisition operation for a Z correction value of head movement;
Fig. 8 is a diagram illustrating an example of an acquisition operation for a Z correction value of head movement;
Fig. 9 is a diagram illustrating an example of an acquisition operation for a Z correction value of head movement;
Fig. 10 is a diagram illustrating an example of an acquisition operation for a Z correction value of head movement;
Fig. 11 is a diagram illustrating an example of an acquisition operation for a Z correction value of head movement;
Fig. 12 is a diagram illustrating an example of an acquisition operation for a Z correction value of head movement;
Fig. 13 is a diagram illustrating an example of an acquisition operation for a Z correction value of head movement;
Fig. 14 is a graph illustrating a relationship between a Z direction position of a tip end and a grid area observed in a grid image;
Figs. 15A, 15B, 15C, and 15D are diagrams illustrating specific examples of a deformation checking portion provided on a bottom surface of the well plate;
Fig. 16 is a flowchart illustrating an example of processing of acquiring a Z correction value of head movement;
Fig. 17 is a flowchart illustrating an example of cell picking processing including the processing of acquiring a Z correction value of head movement; and
Fig. 18 is a flowchart illustrating another example of the cell picking processing including the processing of acquiring a Z correction value of head movement.

### DETAILED DESCRIPTION

Hereinafter, an embodiment of the present invention will be described in detail with reference to the drawings. The cell handling apparatus according to the present invention can suck, transfer, and discharge various biologically derived cells. Examples of the biologically derived cells include cells such as a blood cell, a dissociated cell, and a fertilized egg, small tissue pieces such as histoculture, cell aggregates such as a spheroid and an organoid, organisms such as a zebrafish and a nematode, and a 2D or 3D cell colony. In the present specification, the term "cell" includes these various cells. In particular, the cell handling apparatus of the present invention is suitable for suction, transfer, and discharge of cells such as a single cell, a cell aggregate, and a cell colony, which generally require picking under microscopic observation.

### [Overall configuration of cell handling apparatus]

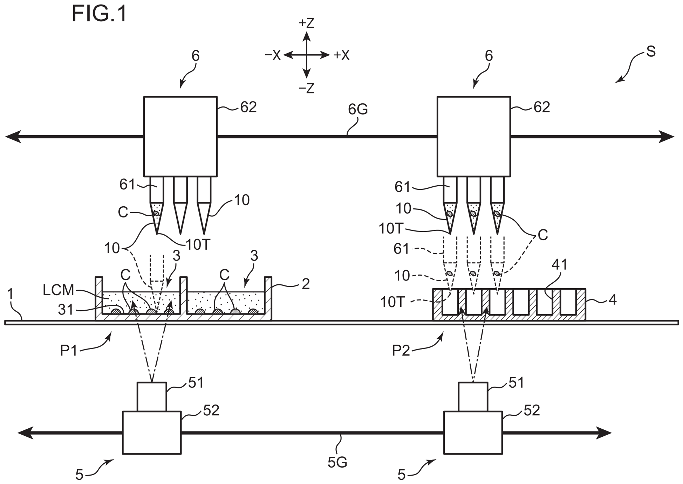

Fig. 1 is a diagram schematically illustrating an overall configuration of a cell handling apparatus S according to an embodiment of the present invention. Here, the cell handling apparatus S for transferring a cell C between two containers, that is, between a first well plate 2 for culturing the cell C and the second well plate 4 as a transfer destination of the cell C is exemplified. As a matter of course, the cell handling apparatus S may be configured to transfer the cell C between three or more containers.

The cell handling apparatus S includes a translucent base 1 having a horizontal placement surface, a camera unit 5 disposed below the base 1, and a head unit 6 disposed above the base 1. The first well plate 2 is placed at a first placement position P1 of the base 1. The second well plate 4 is placed at a second placement position P2. The head unit 6 includes a plurality of heads 61 that can be raised and lowered in a Z direction which is a vertical direction. A tip 10 for sucking and discharging the cell C is attached to a lower end of each of the heads 61. The camera unit 5 and the head unit 6 are movable in an X direction which is a horizontal direction and a Y direction perpendicular to the plane of the drawing of Fig. 1. The well plates 2 and 4 are placed on an upper surface of the base 1 within a movable range of the head unit 6.

Generally, the cell handling apparatus S performs picking for individually sucking a specific target cell C with each of a plurality of the tips 10 from the first well plate 2 in which a large number of the cells C are cultured. Next, the cell handling apparatus S transfers the picked cell C to the second well plate 4, and discharges the cell C from a plurality of the tips 10 toward the well plate 4 (a well 41). Before picking of the cell C, the cell C held in the first well plate 2 is imaged by the camera unit 5, and a selection operation in which the target cell C to be transferred to the second well plate 4 is selected is performed.

Hereinafter, each part of the cell handling apparatus S will be described. The base 1 is a flat plate that has predetermined rigidity and is partially or entirely made from a translucent material. The preferred base 1 is a glass plate. By forming the base 1 with a translucent material such as a glass plate, the camera unit 5 disposed below the base 1 can image the well plates 2 and 4 disposed on an upper surface of the base 1 through the base 1.

The first well plate 2 is a container as a transfer source of the cell C, and includes a plurality of wells 3 for culturing the cell C. Each of the wells 3 is a small open top container, and has a flat bottom surface 31. The cultured cell C is held on (in contact with or suspended on) the bottom surface 31. Into the well 3, a liquid medium LCM is injected, and a large number of the cells C are seeded. For the first well plate 2, a member made from a translucent resin material or glass is used in order to enable imaging of the cell C by the camera unit 5 disposed below. As the first well plate 2, for example, a commercially available 6-well plate (for example, model number 3516 manufactured by Corning Incorporated) can be used.

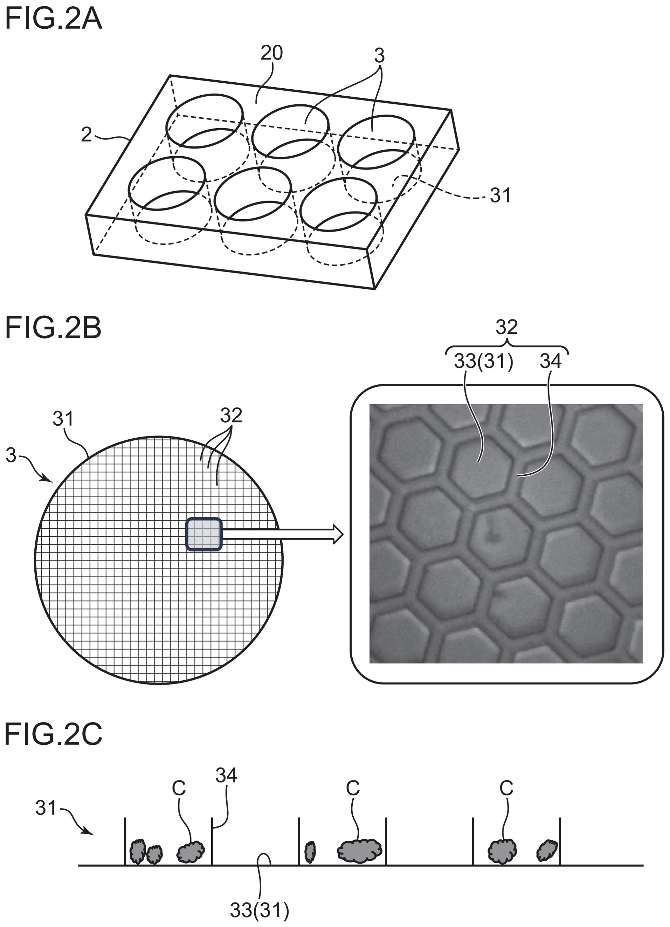

A specific example of the first well plate 2 is shown. Fig. 2A is a perspective view illustrating a 6-well plate as an example of the well plate 2. The well plate 2 includes a plate-like plate body 20 and a plurality of the wells 3. The well 3 is a cylindrical bottomed hole formed in the plate body 20. The wells 3 arranged in two rows and three columns are provided in a plate body 20.

Fig. 2B is a plan view of one of the wells 3, and illustrates the bottom surface 31 of the well. A large number of grids 32 are regularly arranged on the bottom surface 31. The grid 32 is a culture space for the cell C. As illustrated in an enlarged view on the right side of Fig. 2B, the grid 32 is formed by a hexagonal grid bottom surface 33 and a partition wall 34 surrounding a periphery of the grid bottom surface 33. Fig. 2B illustrates a plurality of the grids 32 arranged in a honeycomb type arrangement pattern. The grid 32 may have another shape, for example, a rectangular shape such as a square or a rectangle, or the bottom surface 31 may have the rectangular grids 32 arranged in a matrix.

The bottom surface 31 has translucency and can be deformed in shape. That is, the bottom surface 31 has translucency so that the cell C held on the bottom surface 31 and a tip end portion 10T of the tip 10 can be visually recognized or imaged in a case where the well plate 2 is observed from the lower surface side. Further, the bottom surface 31 has flexibility such that the shape is changed by elastic deformation in a case where the bottom surface 31 is pressed by the tip 10. The bottom surface 31 can be formed of, for example, a transparent resin sheet having a large number of the grids 32 arranged in a predetermined pattern.

Fig. 2C is a longitudinal cross-sectional view of the grid 32. The grid bottom surface 33 is a substantially horizontal surface. The grid bottom surface 33 is also the bottom surface 31 of the well 3. The partition wall 34 is erected vertically upward from the grid bottom surface 33. One or a plurality of the cells C are contained in one of the grids 32. As a matter of course, the grid 32 in which none of the cells C is contained as a result of seeding also occurs. One of the grids 32 serves as a suction or discharge unit of the cell C by the tip 10. That is, the tip end portion 10T of the tip 10 approaches from above with respect to one of the grids 32, and the cell C is sucked or discharged. In the present embodiment, the well 3 or the well plate 2 including the bottom surface 31 having a large number of the grids 32 corresponds to a "container" in the present invention. Further, an example in which a state checking portion for recognizing a shape change of the bottom surface 31 is a shape of the grid 32 itself or an arrangement pattern of the grids 32 is illustrated. The present embodiment mainly shows an example in which the state checking portion is a shape checking portion for checking a shape change of the bottom surface 31 due to contact of a tip end of the tip 10 with the bottom surface 31.

The second well plate 4 has a plurality of the wells 41 to which the cell C picked from the first well plate 2 is discharged. The well 41 is a bottomed hole opened in an upper surface of the well plate 4. One of the wells 41 contains a required number of the cells C together with a liquid medium. The required number is usually one. Various tests such as addition of a reagent or a reactant, observation, culture, and the like are performed on the cell C contained in the well 41. A member made from a translucent resin material or glass is also used as the well plate 4. As the well plate 4, for example, a commercially available 96-well plate (for example, model number 3595 manufactured by Corning Incorporated) can be used.

The camera unit 5 captures images of the cells C held in the well plates 2 and 4 from the lower surface side of the well plates. Specifically, the camera unit 5 images the cell C on the bottom surface 31 and the tip end portion 10T of the tip 10 by imaging the bottom surface 31 of the well 3 from below. The camera unit 5 includes a lens unit 51 and a camera body 52. The lens unit 51 is an objective lens used in an optical microscope and includes a lens group that forms an optical image of a predetermined magnification and a lens barrel that contains the lens group. The camera body 52 includes an imaging element such as a CCD image sensor. The lens unit 51 forms an optical image of an imaging object on a light receiving surface of the imaging element. The camera unit 5 is movable in the X direction and the Y direction below the base 1 along a guide rail 5G extending in a left-right direction in parallel with the base 1. Further, the lens unit 51 is movable in the Z direction for focusing operation.

The head unit 6 sucks the cell C from the first well plate 2 and transfers the cell C to the second well plate 4. The head unit 6 includes a plurality of the heads 61 and a head body 62 to which the heads 61 are assembled. The tip 10 for sucking and discharging the cell C is attached to a tip end of each of the heads 61. The head body 62 holds the head 61 so as to be raised and lowered in +Z and -Z directions which are vertical directions. The head body 62 is movable in +X and -X directions which are horizontal directions along a guide rail 6G. Note that the head body 62 is also movable in the Y direction. That is, the head 61 is movable in three-dimensional directions of X, Y, and Z.

A general operation of the head 61 is as follows. The head 61 on which the tip 10 is mounted is XY-moved to a coordinate position corresponding to a position of the target cell C in the well 3 as a transfer target. The target cell C is identified based on an image of the first well plate 2 captured by the camera unit 5. Subsequently, the head 61 is lowered so as to approach the target cell C from above. After the approach, the target cell C is sucked into the tip 10. After the above, the head 61 is raised and XY-moved to the second well plate 4. The target cells C in the tip 10 are discharged to a predetermined one of the wells 41.

### [Details of tip and head]

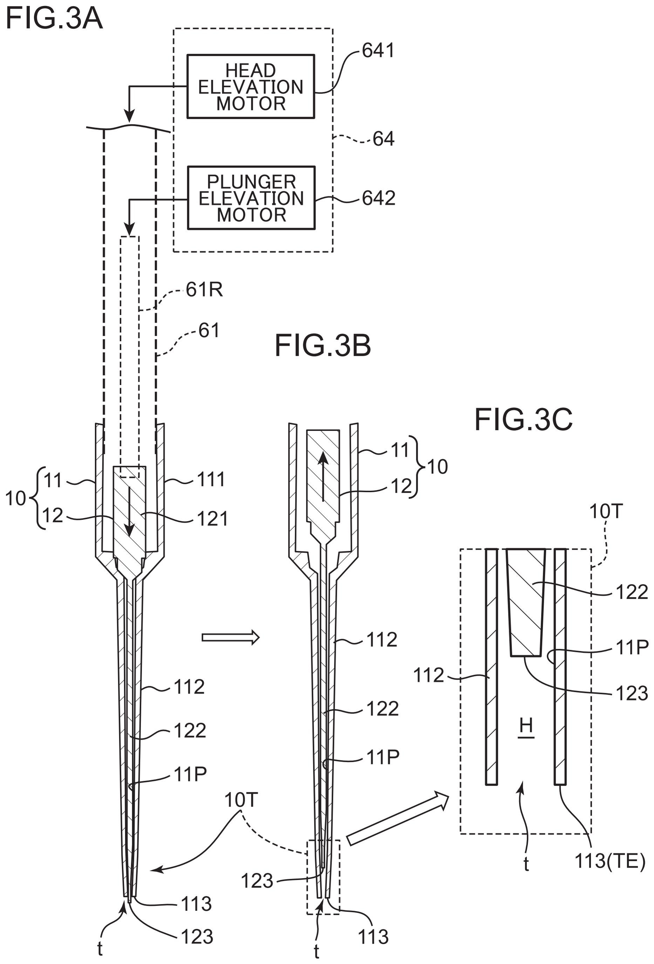

Fig. 3A is a diagram illustrating a cross-sectional view of the tip 10 attached to the head 61, and a moving mechanism and a suction mechanism of the tip 10. The tip 10 is a tool that sucks or discharges the cell C for transfer of the cell C. The tip 10 includes the tip end portion 10T having a tip opening t through which the cell C can enter and exit. The tip 10 includes an assembly of a syringe 11 and a plunger 12. The syringe 11 includes a tubular passage 11P serving as a suction path of the cell C in the inside. The plunger 12 moves forward and backward in the tubular passage 11P while being in sliding contact with an inner peripheral wall of the syringe 11 forming the tubular passage 11P.

The syringe 11 includes a syringe base end portion 111 formed of a cylindrical body having a large diameter and a syringe body portion 112 formed of a long cylindrical body having a small diameter. The tubular passage 11P is formed in the syringe body portion 112. The above-described tip opening t is provided in a syringe tip end portion 113 which is a lower end of the syringe body portion 112. One end of the tubular passage 11P is connected to the tip opening t. The syringe base end portion 111 is connected to another end side of the syringe body portion 112 via a tapered portion. An upper end portion of the syringe base end portion 111 is fitted to a lower end of the head 61.

The plunger 12 includes a plunger base end portion 121 including a cylindrical body, a needle-shaped plunger body portion 122 continuous below the plunger base end portion 121, and a plunger tip end portion 123 which is a lower end of the plunger body portion 122. The plunger 12 is assembled to the syringe 11 in a mode in which the plunger base end portion 121 is contained in the syringe base end portion 111 and the plunger body portion 122 is inserted through the tubular passage 11P of the syringe body portion 112. Fig. 3A illustrates a state in which the plunger body portion 122 is inserted through the syringe body portion 112 to a deepest position. In this state, the plunger tip end portion 123 protrudes from the tip opening t. A rod 61R movable in the vertical direction in an internal space of the head 61 is attached to an upper end of the plunger base end portion 121.

The head body 62 is equipped with a head drive unit 64. The head drive unit 64 functions as a mechanism for moving the tip 10 attached to the head 61 in the vertical direction and a mechanism for sucking and discharging the cell C into and from the inside of the tip 10 through the tip opening t of the tip 10. The head drive unit 64 includes a head elevation motor 641 and a plunger elevation motor 642.

The head elevation motor 641 is a drive source that raises and lowers the head 61 with respect to the head body 62. When the head 61 is raised and lowered by driving of the head elevation motor 641, the tip 10 attached to a lower end of the head 61 is also raised and lowered. That is, a height position of the tip opening t of the tip end portion 10T can be set to a desired position by operation control of the head elevation motor 641.

The plunger elevation motor 642 serves as a drive source that raises and lowers the rod 61R in an internal space of the head 61. When the rod 61R is raised and lowered, the plunger 12 attached to the rod 61R is also raised and lowered. When the plunger 12 is raised with respect to the syringe 11, suction force is generated at the tip opening t. On the other hand, when the plunger 12 is lowered with respect to the syringe 11, discharge force is generated at the tip opening t. That is, a suction operation and a discharge operation of the cell C by the tip 10 can be controlled by operation control of the plunger elevation motor 642.

Fig. 3A illustrates a state in which the plunger 12 is lowered to its lowest position. This state is a state before the cell C is sucked or a state in which the cell C sucked to the tip 10 is discharged. The plunger tip end portion 123 protrudes slightly downward from the syringe tip end portion 113. Fig. 3B illustrates a state in which the plunger 12 is raised by a predetermined height. This state is a state of the tip 10 at the time of a suction operation of sucking the cell C. Fig. 3C is an enlarged diagram of a main part of Fig. 3B. The syringe tip end portion 113 in the present embodiment corresponds to an "tip end of a tip" of the present invention. As additionally illustrated in Fig. 3C, in the following description, the syringe tip end portion 113 may be referred to as a tip end TE to mean a tip end of the tip 10.

During a suction operation, the plunger tip end portion 123 retracts into the tubular passage 11P. At this time, suction force is generated at the tip opening t, and fluid around the tip opening t is sucked into a suction space H formed in the tubular passage 11P as the plunger tip end portion 123 retracts inward. That is, the medium LCM containing the cell C is held in the suction space H. After this suction operation, when the plunger 12 is moved downward, the fluid held in the suction space H is discharged from the tip opening t. A suction amount of the fluid can be adjusted by a raised height of the plunger 12, and a suction speed of the fluid can be adjusted by a raising speed of the plunger 12.

### [Electrical configuration of cell handling apparatus]

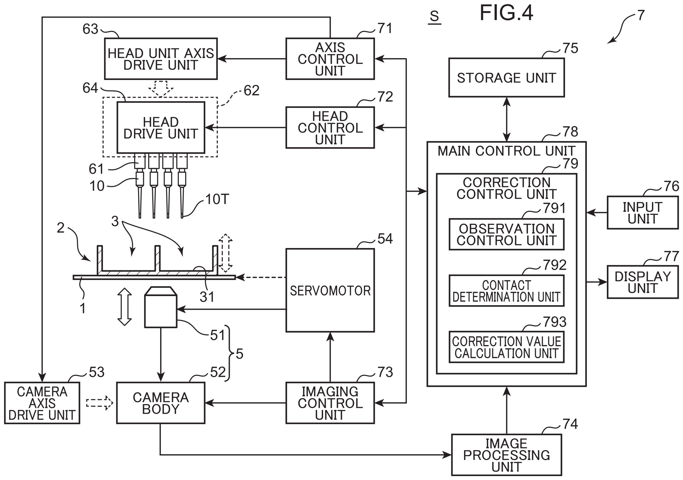

Fig. 4 is a block diagram illustrating an electrical configuration of the cell handling apparatus S. The cell handling apparatus S includes a control unit 7 that controls horizontal movement of the head unit 6 (see Fig. 1) in the XY directions and raising and lowering of the head 61 (tip 10) in the Z direction, that is, movement in three-dimensional directions of the head 61. In addition, the control unit 7 controls suction and discharge operations of the cell C to and from the tip 10, and movement, an imaging operation, and the like of the camera unit 5.

The cell handling apparatus S includes a camera axis drive unit 53, a servomotor 54, a head unit axis drive unit 63, and the head drive unit 64. The camera axis drive unit 53 includes a drive motor that horizontally moves the camera unit 5 along the guide rail 5G (Fig. 1). The servomotor 54 rotates forward or backward to move the lens unit 51 in the vertical direction with predetermined resolution via a power transmission mechanism (not illustrated). By this, a focal position of the lens unit 51 is aligned with the cell C contained in the well 3. Note that, as indicated by a dotted line in Fig. 4, not the lens unit 51 but the base 1 side may be moved up and down. The head unit axis drive unit 63 includes a drive motor that horizontally moves the head unit 6 in the X or Y direction along the guide rail 6G. The head drive unit 64 is as described above with reference to Fig. 3.

The control unit 7 includes a processor and the like, and functions to include an axis control unit 71, a head control unit 72, an imaging control unit 73, an image processing unit 74, a storage unit 75, and a main control unit 78 by execution of a predetermined program. Furthermore, an input unit 76 that inputs various types of information to the control unit 7 and a display unit 77 that displays various types of information are provided. The input unit 76 receives input of various types of operation information from an operator. The display unit 77 functions as a monitor that displays an image and the like captured by the camera unit 5.

The axis control unit 71 controls an operation of the head unit axis drive unit 63. The axis control unit 71 moves the head unit 6 to a predetermined target position in the horizontal direction by controlling the head unit axis drive unit 63. Movement of the head 61 (tip 10) between the well plates 2 and 4, positioning of the head 61 (tip 10) vertically above with respect to the cell C in the well 3, positioning of the head 61 (tip 10) vertically above with respect to the well 41 as a cell discharge target, and the like are realized by the axis control unit 71 controlling the head unit axis drive unit 63. Further, the axis control unit 71 also controls the camera axis drive unit 53 to control an operation of moving the camera unit 5 along the guide rail 5G. Furthermore, an operation of the head 61 for bringing the tip 10 into contact with the bottom surface 31 of the well 3 to deform the bottom surface 31, which is a main point of the present embodiment, is also controlled by the axis control unit 71.

The head control unit 72 controls the head elevation motor 641 of the head drive unit 64 to raise or lower the head 61 to be controlled toward a predetermined target position. Further, the head control unit 72 generates suction force or discharge force at the tip opening t of the tip 10 at a predetermined timing by controlling the plunger elevation motor 642.

The imaging control unit 73 controls an imaging operation of the first well plate 2 or the second well plate 4 by the camera unit 5, for example, an exposure amount, a shutter timing, and the like. Furthermore, the imaging control unit 73 gives a control pulse for moving the lens unit 51 in the vertical direction at a predetermined pitch to the servomotor 54 for a focusing operation for imaging the cell C, detection of the tip end TE of the tip, recognition of shape deformation of the grid 32 arranged on the bottom surface 31 of the well 3, and the like. The pitch is, for example, a pitch of tens of micrometers.

The image processing unit 74 performs image processing such as edge detection processing and pattern recognition processing accompanied by feature amount extraction on image data acquired by the camera body 52. Based on an image of the first well plate 2 on which the cell C is cultured, the image processing unit 74 executes processing of recognizing, on the image, presence of the cell C on the bottom surface 31 and shape deformation of the grid 32 on the bottom surface 31 caused by pressing of the tip 10. Similarly, the image processing unit 74 executes processing of recognizing the number, amount, fluorescence intensity, and the like of the cell C contained in the well 41 based on an image of the well 41 of the second well plate 4 to which the cell C is transferred.

The storage unit 75 stores various setting values, data, programs, and the like in the cell handling apparatus S. In addition, the storage unit 75 stores information regarding the well plates 2 and 4 to be used, for example, data such as a plate size, sizes of the well 3 and the grid 32, an arrangement pattern of the grids 32, and information on irregularities of the bottom surface 31. Further, information regarding a tip shape of the tip 10, for example, an outer diameter and thickness of the syringe tip end portion 113, an opening diameter of the tip opening t, and the like are also stored in the storage unit 75. Furthermore, setting information such as a suction amount and a suction speed in a suction operation of the cell C, and a moving pitch and a horizontal moving amount in the Z direction of the head 61 in pressing control of the tip 10 against the bottom surface 31 are also stored in the storage unit 75.

The main control unit 78 integrally controls operations of the camera unit 5 and the head unit 6. The main control unit 78 controls the camera unit 5 and the head unit 6 through the axis control unit 71, the head control unit 72, and the imaging control unit 73 so that imaging of the first well plate 2, picking of causing the tip 10 to suck the target cell C, and transfer of the target cell C to the second well plate 4 are executed. Furthermore, the main control unit 78 of the present embodiment controls correction processing for correcting position coordinates of the head 61. The correction processing includes an operation of bringing the tip 10 attached to the head 61 into contact with the bottom surface 31 of the well 3 to deform the grid 32 as a deformation checking portion.

The main control unit 78 functionally includes a correction control unit 79 for the above correction control. The correction control unit 79 includes an observation control unit 791, a contact determination unit 792, and a correction value calculation unit 793.

The observation control unit 791 performs processing of designating a target grid and a shape observation grid from among a large number of the grids 32 arranged on the bottom surface 31 and processing of setting an elevation sequence of the head 61. The target grid is a grid with which the tip end TE is brought into contact. The shape observation grid is a grid that is located around the target grid for observation of a shape change based on contact of the tip end TE in an image. The elevation sequence is elevation control of the head 61 including lowering of the tip end TE, contact with the target grid, a pressing operation against the bottom surface 31 after contact, and elevation of the tip end TE.

The contact determination unit 792 detects the contact of the tip end TE with the bottom surface 31 based on a shape change of the grid 32 or an arrangement pattern of the grid 32. Specifically, the contact determination unit 792 performs detection based on a shape change of a shape observation grid as a deformation checking portion in an image of the bottom surface 31 captured by the camera unit 5 in the correction processing. The contact determination unit 792 determines that the tip end TE comes into contact with the bottom surface 31 when a shape change of the shape observation grid exceeds a predetermined threshold.

For example, the contact determination unit 792 observes an area of the shape observation grid on a two-dimensional image, and determines that the tip end TE comes into contact with the bottom surface 31 when the area becomes smaller than a predetermined threshold. Instead of the area, a contour shape on a two-dimensional image of the shape observation grid may be observed, and when a change rate of the contour shape becomes larger than a predetermined threshold, it may be determined that the tip end TE comes into contact with the bottom surface 31. Alternatively, contact of the tip end TE with the bottom surface 31 may be detected based on a state change such as a change in a luminance value without depending on a shape change on a two-dimensional image. In this case, a change in a luminance value is observed, and it is determined that the tip end TE comes into contact with the bottom surface 31 when a change rate of the luminance value becomes larger than a predetermined threshold. In a portion pressed by the tip end TE on the bottom surface 31, a state change such as that the tip end TE is brightened by illumination light or the grid 32 is inclined and darkened can be observed in a two-dimensional image. Such a state change can be regarded as a change in a luminance value.

The correction value calculation unit 793 acquires a correction value of position coordinates of the head 61 by obtaining a difference between a lowering target position of the tip end TE and an actual lowering position where the tip end TE is actually in contact with the bottom surface 31. The lowering target position is a movement target position of the tip end TE given as a control value, and is set based on, for example, a height position of the tip end TE based on a design value or the like or a theoretical height position of the bottom surface 31. The actual lowering position is a position where the head 61 is actually lowered and the tip end TE reaches the bottom surface 31. A theoretical position of the tip end TE and an actual position may deviate from each other due to various factors. The correction value calculation unit 793 derives a correction value in movement control of the head 61 that corrects the deviation based on a difference between both of the positions.

### [Problems in cell picking]

In order to accurately suck, that is, pick the target cell C with the tip 10, it is important to accurately align the tip opening t of the tip 10 with the target cell. However, in actual cell picking, even if the head 61 is moved to target position coordinates, the picking of the target cell C may fail. Examples of the factors that inhibit the alignment include positional misalignment of the tip end TE in a state of being attached to the head 61 and a height variation of the bottom surface 31 of the well 3 that is a culture surface of the cell C. The same applies to a case of discharging the cell C to a predetermined target position.

The positional misalignment of the tip end TE is caused by attaching positional misalignment of the tip 10 with respect to the head 61, thermal expansion and contraction of the guide rail 6G which is a moving axis of the head 61, other mechanical errors, a range of depth of focus in a case where a height position of the tip end portion 10T is identified based on a camera image, and the like. As illustrated in Fig. 3A, the tip 10 is fitted into a lower end of the head 61, but a fitting error inevitably occurs. There is also a dimensional error of the tip 10 itself. Further, influence of thermal expansion and contraction, a mechanical error, and depth of focus cannot be reduced to zero. That is, a position of the tip end TE is misaligned in the XYZ directions.

If there is positional misalignment in the XY directions in the tip end TE, even if the head 61 is moved so as to direct the tip end TE toward a target coordinate position, an actual movement position of the tip end TE is misaligned relatively to the XY position immediately above the target cell C. If there is positional misalignment in the Z direction in the tip end TE, an error occurs in approach height to the target cell C. In such a situation, picking of the target cell C by the tip 10 may fail.

In addition to the above, the bottom surface 31 of the well 3 has non-negligible variation in height, that is, surface irregularities. Thickness size of the cell C to be picked is, for example, about several micrometers to several tens of micrometers. The bottom surface 31 is a macroscopically horizontal surface, but when observed on the order of micrometers, surface irregularities on the order of several micrometers to several tens of micrometers exist. As a result of measurement by the present inventors, it was confirmed that, for the height of the bottom surface 31 of the well 3 of a commercially available 6-well plate, a height difference of about 100 µm at the maximum existed between six wells. In a case where there is such variation in the height of the bottom surface 31, the cell C may not be sucked even if the tip end TE is lowered to a certain height position.

### [Control of acquisition of movement correction value of head]

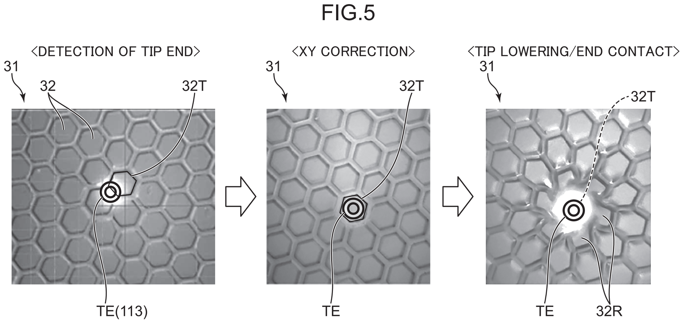

In order to correct positional misalignment of the tip end TE in the XYZ directions, the correction control unit 79 causes the camera unit 5 and the head unit 6 to execute an operation for acquiring a movement correction value of the head 61. Fig. 5 is a diagram illustrating an outline of the movement correction operation of a head. As described above, the movement correction operation includes an operation of bringing the tip end TE into contact with a target grid 32T. Fig. 5 is an image obtained by the camera unit 5 capturing the bottom surface 31 of the well 3 from below, and images sequentially captured as the tip 10 is lowered are arranged. In the image, the grids 32 arranged in a honeycomb shape are shown.

When the head 61 is lowered, the tip end TE eventually appears in an image captured by the camera unit 5. The image processing unit 74 performs image recognition processing of the tip end TE on the image, so that the tip end TE is detected. By the detection, positional misalignment in the horizontal direction between a position of the target grid 32T that is intended to be in contact with the tip end TE and a current position of the tip end TE is detected. Next, the head 61 is moved in the XY directions so as to correct the positional misalignment in the horizontal direction. The central diagram of Fig. 5 illustrates a state in which XY correction of the head 61 is completed, the target grid 32T and the tip end TE are aligned, and the tip end TE is lowered until the tip end TE is focused. A focus position of the lens unit 51 of the camera unit 5 is set near the bottom surface 31. For this reason, when the tip end TE is clearly observed, it can be estimated that the tip end TE is located at or in the vicinity of a position in contact with the bottom surface 31. However, it is not possible to determine whether or not the tip end TE is actually in contact with the grid bottom surface 33 of the target grid 32T, that is, the bottom surface 31.

After the above, the head 61 is further lowered. As described above, the bottom surface 31 has flexibility that allows its shape to be changed by elastic deformation. Therefore, the bottom surface 31 is deformed so as to be recessed downward by contact of the tip end TE and pressing force after the contact. The deformation of the bottom surface 31 causes shape deformation of the grid 32 or an arrangement pattern of the grids 32. That is, as illustrated in the right diagram of Fig. 5, when the target grid 32T is pressed by the tip end TE, an adjacent grid 32R around the target grid 32T is deformed in a stretched manner. By observing such deformation of the adjacent grid 32R on an image, it is possible to find contact of the tip end TE with the bottom surface 31.

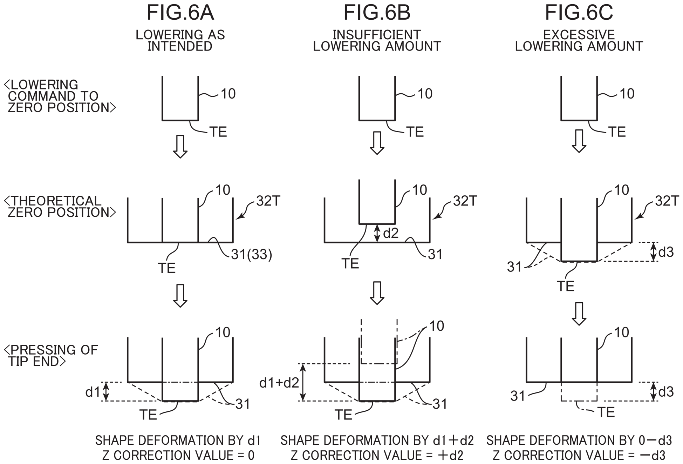

Figs. 6A, 6B, and 6C are diagrams illustrating a basic idea of acquiring a movement correction value of the head 61. The correction value calculation unit 793 acquires the movement correction value by obtaining a difference between a lowering target position of the tip end TE and an actual movement position at which the tip end TE comes into contact with the bottom surface when the head 61 is lowered so that the tip end TE is directed toward a certain target position. In the following description, a case where the lowering target position is the bottom surface 31, that is, a case where a height position of the tip end TE is a "zero position" is assumed.

Fig. 6A illustrates an example in which the tip end TE is lowered as intended, that is, an example in which positional misalignment of the tip end TE in the Z direction is zero. The head unit axis drive unit 63 controls an operation of the head drive unit 64 so that the head 61 is lowered according to a lowering target position given from the axis control unit 71. If there is no positional misalignment in the tip end TE, the tip end TE comes into contact with a theoretical zero position, that is, the bottom surface 31. However, even if the contact is made, the contact cannot be definitively determined from an image captured by the camera unit 5. For this reason, the head 61 is further lowered by a distance d1. By lowering by the distance d1, the tip end TE presses the bottom surface 31 of the target grid 32T and deforms the bottom surface so as to be recessed downward. The distance d1 is any distance required to make a shape change of the bottom surface 31 recognizable after the contact of the tip end TE with the bottom surface 31. In this case, since it is confirmed that the lowering target position of the tip end TE = the actual movement position, the movement correction value in the Z direction = 0.

Fig. 6B illustrates an example in which a lowering amount of the tip end TE is insufficient. That is, this is an example in which even if the head 61 is lowered based on a lowering target position, an actual movement position of the tip end TE is at a position above the bottom surface 31 which is the theoretical zero position by a distance d2. In this case, the tip end TE does not come into contact with the bottom surface 31 unless the head 61 is lowered by the distance d2, and a shape of the bottom surface 31 does not change unless the head 61 is further lowered by the distance d1. Therefore, in the case of Fig. 6B, the movement correction value = +d2.

Fig. 6C illustrates an example in which a lowering amount of the tip end TE is excessive. That is, this is an example in which, when the head 61 is lowered based on a lowering target position, an actual movement position of the tip end TE is at a position lower than the bottom surface 31 by a distance d3. In this case, the tip end TE comes into contact with the bottom surface 31 only by lowering the head 61 to a position higher than the lowering target position by the distance d3. Therefore, in the case of Fig. 6C, the movement correction value = -d3.

### [Example of determination of contact of tip end with bottom surface]

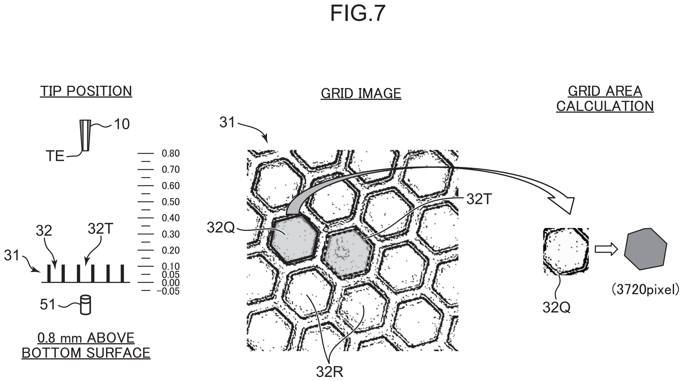

Figs. 7 to 13 are diagrams illustrating examples of determination of contact of the tip end TE with the bottom surface 31 in an operation of acquiring a movement correction value in the Z direction of the head 61. In Figs. 7 to 13, the left column shows a tip position, which is a position of the tip end TE of the tip 10 with respect to the bottom surface 31 of the well 3 in which the grid 32 is arranged. The middle column shows a grid image after image processing such as contrast enhancement, edge detection, and binarization processing is performed on an image obtained by the camera unit 5 imaging the bottom surface 31 through the lens unit 51 at the tip position. One of six of the adjacent grids 32R of the hexagonal target grid 32T is designated as a shape observation grid 32Q as a deformation checking portion. The right column shows a shape of a single body of the shape observation grid 32Q and a calculation result of a grid area of the shape observation grid 32Q. The grid area is derived by the number of pixels existing inside a contour line of the shape observation grid 32Q extracted by the image processing.

Fig. 7 illustrates a state in which the tip position is 0.8 mm above the bottom surface 31. The tip end TE does not appear yet in the grid image. Since the bottom surface 31 is not pressed by the tip end TE, no remarkable shape change appears in the shape observation grid 32Q in the grid image. In grid area calculation, an area of the shape observation grid 32Q is calculated to be 3720 pixels.

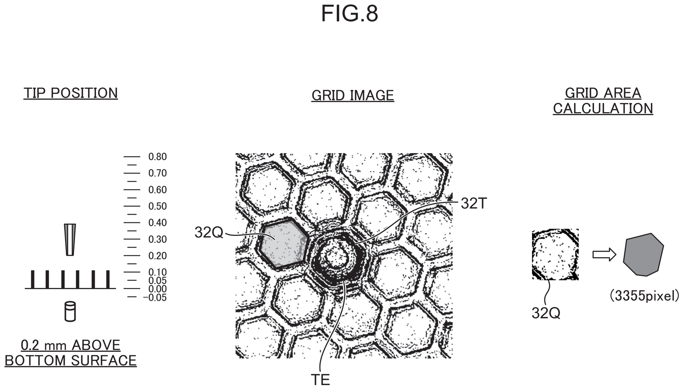

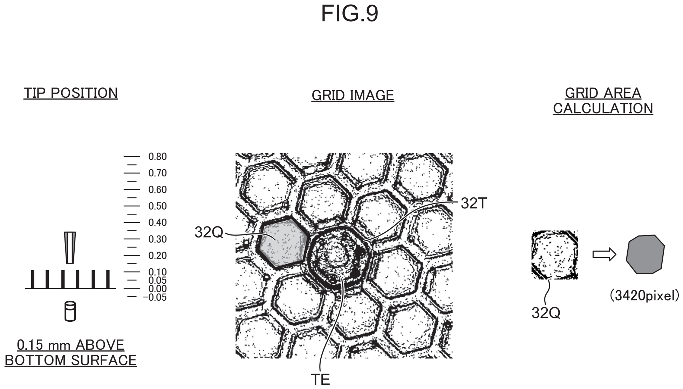

Fig. 8 illustrates a state in which the tip position is 0.2 mm above the bottom surface 31. The tip end TE appears in the grid image, but is not focused. No substantial shape change occurs in the shape observation grid 32Q, and a calculated value of 3355 pixels is output in the grid area calculation. Fig. 9 illustrates a state in which the tip position is 0.15 mm above the bottom surface 31. The tip end TE that is not focused is observed in the grid image, and a calculated value of 3420 pixels is output in the grid area calculation.

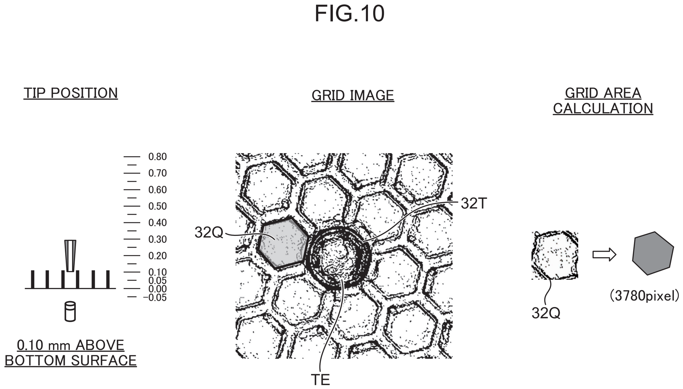

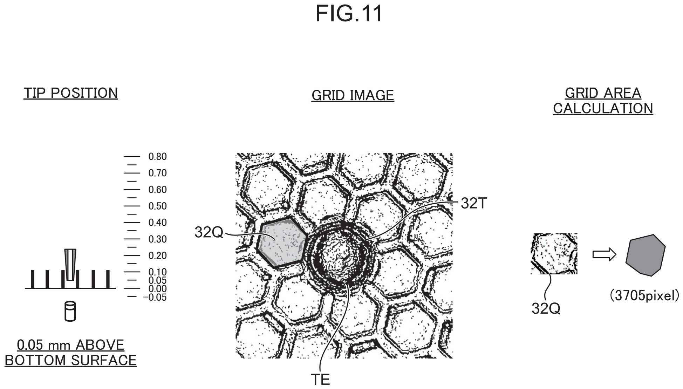

Fig. 10 illustrates a state in which the tip position is 0.10 mm above the bottom surface 31, and Fig. 11 illustrates a state in which the tip position is 0.05 mm above the bottom surface 31. When the tip end TE is lowered to this point, the tip end TE enters the target grid 32T. A focus position of the lens unit 51 is set to the bottom surface 31, but since there is a depth of field, the tip end TE is observed relatively clearly. Since the tip end TE is not yet in contact with the bottom surface 31, no remarkable shape change is observed in the shape observation grid 32Q in the grid image. In the grid area calculation, 0.10 mm above = 3780 pixels and 0.05 mm above =3705 pixels are output.

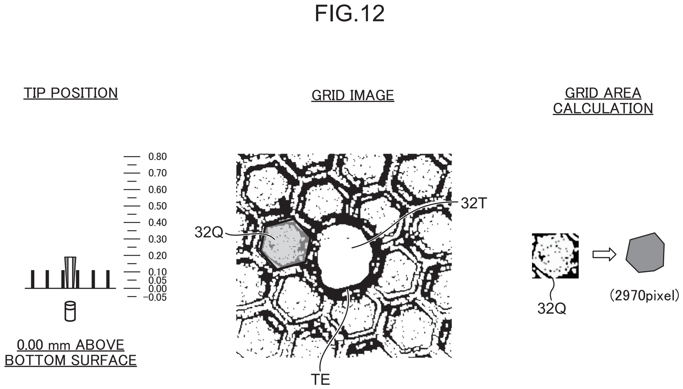

Fig. 12 illustrates a state in which the tip position is 0.00 mm above the bottom surface 31. That is, Fig. 12 illustrates a state in which the tip end TE is in contact with the bottom surface 31 of the target grid 32T. Since the tip 10 is illuminated by illumination light at the time of imaging, the bottom surface 31 portion on which the tip end TE abuts in a grid image is observed to be brighter than the other portions. A slight shape change is observed in the shape observation grid 32Q due to the contact of the tip end TE with the bottom surface 31. This is because a slight pressing force acts on the bottom surface 31 by the contact. In the grid area calculation, a calculated value of 2970 pixels is output. This calculated value can be said to be a significantly small value as compared with the previous calculated values exceeding 3000 pixels.

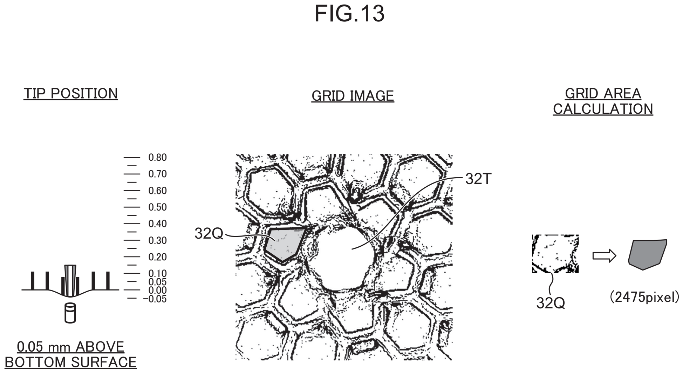

Fig. 13 illustrates a state in which the tip position is -0.05 mm above the bottom surface 31. That is, Fig. 13 illustrates a state in which the tip end TE presses the bottom surface 31 of the target grid 32T and the bottom surface 31 is recessed by 0.05 mm. In the grid image, a significant shape change of the shape observation grid 32Q is observed. Since the tip end TE recesses the bottom surface 31 of the target grid 32T, the adjacent shape observation grid 32Q is pulled downward, and a shape change is caused. In the grid area calculation, a calculated value of 2475 pixels is output, and the area calculated value is significantly reduced.

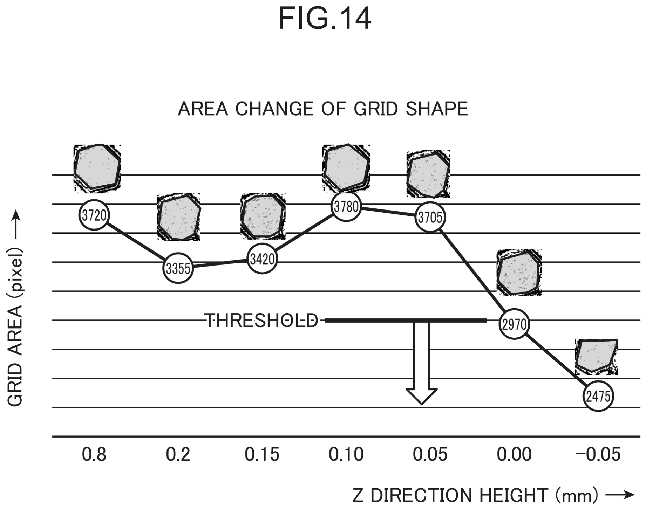

Fig. 14 is a graph illustrating a relationship between a Z direction position of the tip end and a grid area calculated from a grid image. At a Z direction height (0.08 mm to 0.05 mm above) before the tip end TE comes into contact with the bottom surface 31, an average value of area calculated values of the shape observation grid 32Q is about 3600 pixels. On the other hand, after the contact of the tip end TE with the bottom surface 31, an area calculated value of the shape observation grid 32Q is 3000 pixels or less. Therefore, contact of the tip end TE with the bottom surface 31 can be detected by monitoring an area change of the shape observation grid 32Q with a grid area = 3000 pixels as a threshold.

As described above, a contour shape of the shape observation grid 32Q is obtained for calculation of a grid area. The configuration may be such that a change in the contour shape is monitored, and when the change rate becomes larger than a predetermined threshold, the tip end TE is determined to come into contact with the bottom surface 31. In this case, a hexagonal template matching a shape of the grid 32 is set. Then, in a grid image acquired every time the tip end TE is lowered, the template is preferably compared with a contour shape of the shape observation grid 32Q to obtain a change rate of the contour shape.

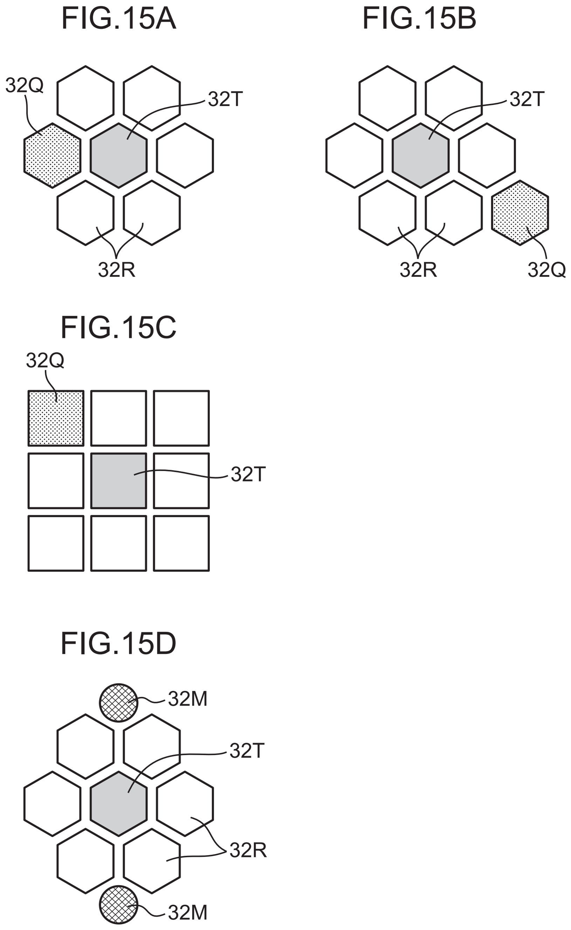

Figs. 15A to 15D are diagrams illustrating specific examples of a deformation checking portion provided on the bottom surface 31 of the well 3. Fig. 15A illustrates an example employed in the examples of Figs. 7 to 13. One of six of the adjacent grids 32R adjacent around the target grid 32T is designated as the shape observation grid 32Q as a deformation checking portion. A plurality of the adjacent grids 32R may be selected as the shape observation grids 32Q. For example, a pair of the adjacent grids 32R facing each other across the target grid 32T may be set as the shape observation grids 32Q.

Fig. 15B illustrates an example in which one of grids located outside the adjacent grid 32R is selected as the shape observation grid 32Q. As described above, the shape observation grid 32Q does not need to be selected from the adjacent grids 32R. Fig. 15C is a diagram illustrating a selection mode of the shape observation grid 32Q in a case of the grid 32 having a rectangular shape. The diagram illustrates an example in which a grid diagonally upper left of the target grid 32T is selected as the shape observation grid 32Q.

The deformation checking portion may be a structural portion other than a grid, or marks. Fig. 15D illustrates an example in which a mark 32M as the deformation checking portion is arranged in the vicinity of the target grid 32T. A pair of the marks 32M are arranged outside the adjacent grid 32R group surrounding the target grid 32T. The mark 32M is paint, a protrusion, or the like attached to the bottom surface 31. When the target grid 32T is pressed by the tip end TE and the bottom surface 31 is recessed and deformed, a shape of the mark 32M also changes. By performing image recognition of a shape change of the mark 32M, it is possible to find contact of the tip end TE with the bottom surface.

### [Processing of acquiring movement correction value]

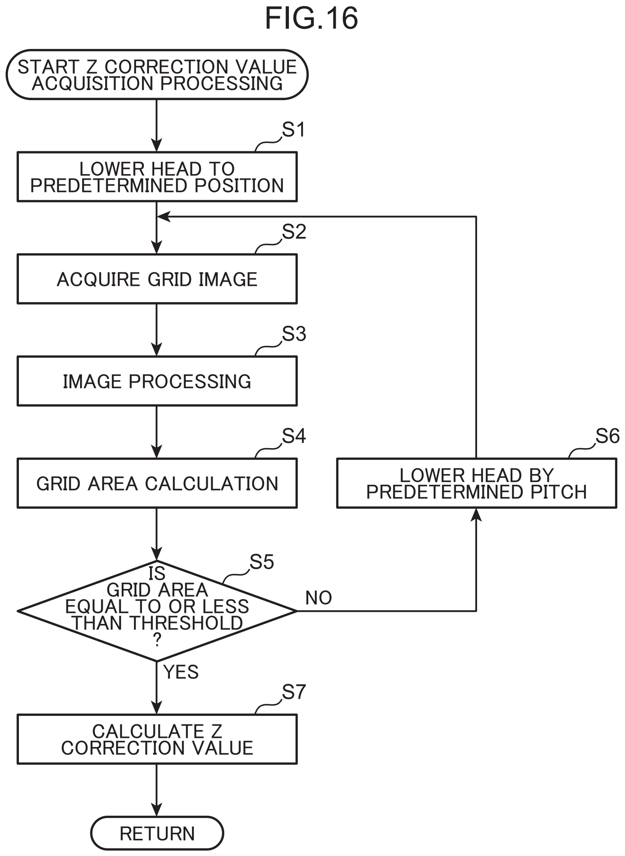

Fig. 16 is a flowchart illustrating an example of processing of acquiring a movement correction value in the Z direction of the head 61. As a premise of start of the processing, it is assumed that movement correction in the XY directions of the head 61 is completed, and alignment of the tip end TE vertically above the target grid 32T is completed. The observation control unit 791 of the correction control unit 79 controls the head drive unit 64 through the head control unit 72 to lower the head 61 to a predetermined position for movement correction in the Z direction of the head 61 (Step S1).

Initial lowering in Step S1 is, for example, lowering until the tip end TE is positioned 0.8 mm above the bottom surface 31 as illustrated in Fig. 7. An initial lowering position is a height position at which the tip end TE enters the well 3 but does not come into contact with the bottom surface 31 even if a mechanical error or a container error is involved. If the head 61 is lowered at a high speed to the initial lowering position, takt time for acquiring a correction value can be shortened. After the initial lowering position, the head 61 is lowered stepwise at a predetermined pitch as described below, and an image of the bottom surface 31 is acquired every one pitch of lowering.

After the head 61 is lowered to a predetermined position, the camera unit 5 images the bottom surface 31 of the well 3 under the control of the imaging control unit 73, and a grid image is acquired (Step S2). The acquired grid image data is sent to the image processing unit 74. The image processing unit 74 performs predetermined image processing on the grid image data (Step S3). The image processing is, for example, contrast enhancement, edge detection, binarization processing, or the like, and is processing for the purpose of clarifying a contour shape of the grid 32 on the bottom surface 31 on the image, as illustrated in Fig. 7 and the like.

Subsequently, the contact determination unit 792 calculates a grid area of the shape observation grid 32Q which is a grid designated as a deformation checking portion (Step S4). As described above, the grid area is derived based on the number of pixels existing inside the contour of the shape observation grid 32Q identified in Step S3. After the above, the contact determination unit 792 determines whether or not the grid area derived in Step S4 is equal to or less than a predetermined threshold (Step S5). In a case where the example of Fig. 14 is applied, the threshold is 3000 pixels.

In a case where the grid area exceeds the predetermined threshold (NO in Step S5), the head control unit 72 lowers the head 61 by a predetermined pitch (for example, 0.05 mm) (Step S6). After the above, the processing returns to Step S2, a grid image is acquired at a position lowered by one pitch, and the processing of Step S3 and subsequent steps is repeated.

In a case where the grid area is equal to or less than the predetermined threshold (YES in Step S5), the contact determination unit 792 determines that the tip end TE comes into contact with the bottom surface 31 of the target grid 32T. As described with reference to Fig. 6, the correction value calculation unit 793 calculates a correction value that brings a Z position where the tip end TE comes into contact to a height zero position (Step S7).

### [Cell picking processing]

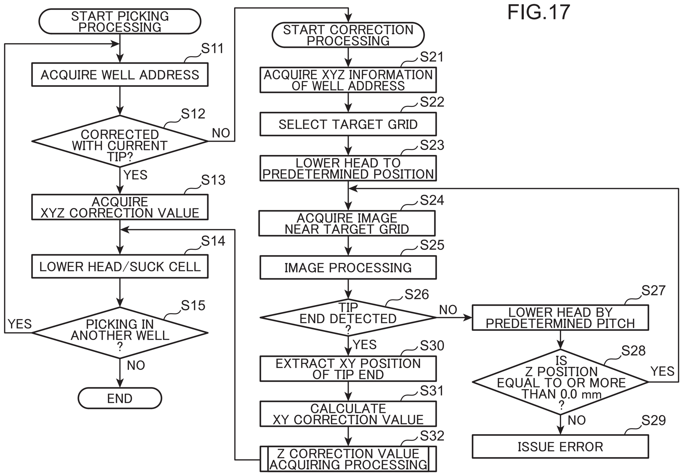

Fig. 17 is a flowchart illustrating an example of cell picking processing including processing of acquiring a movement correction value in the XYZ directions of the head 61. The main control unit 78 acquires address data of the well 3 containing the target cell C to be transferred from the first well plate 2 to the second well plate 4 designated by the user via the input unit 76 (Step S11). The main control unit 78 checks whether or not position correction processing for the tip 10 currently attached to the head 61 is completed for the well 3 of the acquired address (Step S12). Specifically, it is checked whether or not movement correction value data in the XYZ directions of the head 61 for the well 3 and the tip 10 exists in the storage unit 75.

When the position correction processing is not completed (NO in Step S12), correction processing of Step S21 and subsequent steps described later is executed. In a case where the position correction processing is completed (YES in Step S12), the main control unit 78 acquires a movement correction value in the XYZ directions from the storage unit 75 (Step S13). Subsequently, the main control unit 78 gives the movement correction value to the head control unit 72 and causes the head control unit 72 to execute picking of the cell C. The head control unit 72 lowers the head toward the grid 32 containing the target cell C and causes the target cell C to be sucked into the tip 10 (Step S14).

The main control unit 78 determines whether or not a picking command is given for another one of the wells 3 (Step S15). In a case where picking is performed in another one of the wells 3 (YES in Step S15), the processing returns to Step S11 and the processing is repeated. In a case where picking is not performed for another one of the wells 3 (NO in Step S15), the picking processing ends. After the above, the picked cell C is transferred to the second well plate 4.

When the correction processing is started, the observation control unit 791 of the correction control unit 79 acquires XYZ position information for the well 3 at the address designated in Step S11 from the storage unit 75 (Step S21). The position information acquired here is a standard value for the well 3, and does not include shape errors such as height variation of the bottom surface 31 that actually exists. Subsequently, the observation control unit 791 selects the target grid 32T with which the tip end TE is brought into contact from the well 3 at the address (Step S22).

The axis control unit 71 XY-moves the head unit 6 so that the head 61 faces the target grid 32T. After the XY movement, the head control unit 72 lowers the head 61 (Step S23). Here, the lowered position may be a height position at which the tip end TE is expected to start to appear in an image of the bottom surface 31 captured by the camera unit 5. In the examples of Figs. 7 to 13, the tip end TE can be confirmed in an image (Fig. 8) of 0.2 mm above the bottom surface 31. Therefore, a lowering height of the tip end TE in Step S23 can be set to, for example, 0.25 mm above the bottom surface 31.

After the above, the imaging control unit 73 causes the camera unit 5 to acquire an image of the bottom surface 31 near the target grid 32T (Step S24). The image processing unit 74 performs image processing such as contrast enhancement, edge detection, binarization processing, and noise removal processing on the acquired image data (Step S25). By the image processing, contour shapes of the target grid 32T and the grids 32 around the target grid 32T are recognized, and in a case where the tip end TE appears, the position of the tip end TE is recognized.

The observation control unit 791 determines whether or not the tip end TE is detected in the image acquired in Step S24 (Step S26). In a case where the tip end TE is not detected (NO in Step S26), the head control unit 72 lowers the head 61 by a predetermined pitch (for example, 0.05 mm) (Step S27).

Next, it is confirmed whether or not a height position of the tip end TE after the head 61 is lowered is 0.0 mm or more above the bottom surface 31 (Step S28). This is because there is a high possibility that some error occurs because the tip end TE is not recognized on the image at 0.0 mm above the bottom surface 31, that is, at a height position where the tip end TE is expected to abut on the bottom surface 31. As a cause of the error, the tip 10 is out of the head 61, the target grid 32T does not enter a camera's field of view, and the like.

When a height position of the tip end TE is 0.0 mm or more above the bottom surface 31 (YES in Step S28), the processing returns to Step S24, and an image of the bottom surface 31 near the target grid 32T in a state where the tip end TE is lowered by one rank is acquired. In a case of NO in Step S28, error information is issued to the display unit 77 or the like (Step S29).

In a case where the tip end TE is detected in Step S26 (YES in Step S26), the correction value calculation unit 793 extracts an XY coordinate position of the tip end TE (Step S30). Note that the determination of YES in Step S26 is made in a case where the tip end TE is detected in a state nearly in focus so that the position of the tip end TE can be accurately identified. Subsequently, the correction value calculation unit 793 calculates an XY movement correction value of the head 61 based on a difference between a coordinate position of the target grid 32T and an XY coordinate position of the tip end TE extracted in Step S30 (Step S31).

After the above, the Z correction value acquisition processing illustrated in Fig. 16 is executed (Step S32). By execution of Step S32, XYZ position correction values for the tip 10 currently attached to the head 61 are obtained for the well 3 at the address designated in Step S11. Therefore, the processing proceeds to Step S14, and a picking operation of the cell C is executed.

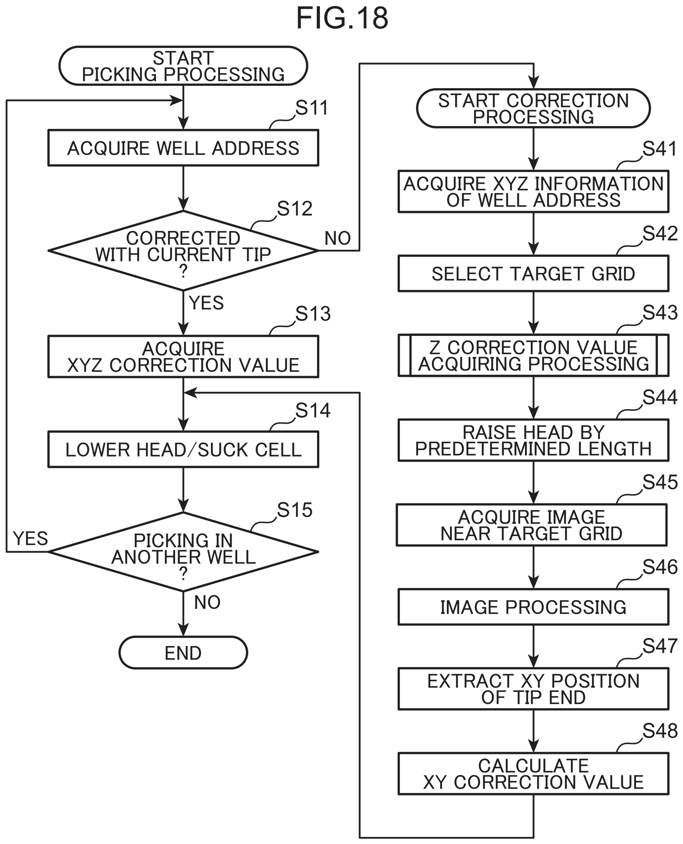

Fig. 18 is a flowchart illustrating another example of cell picking processing including processing of acquiring a movement correction value in the XYZ directions of the head 61. Here, an example in which a Z correction value is acquired earlier than an XY correction value will be described. Since Steps S11 to S15 in Fig. 18 are the same as Steps S11 to S15 described above with reference to Fig. 17, description of the steps will be omitted. In Step S12, in a case where it is determined that the position correction processing for the tip 10 currently attached to the head 61 is not completed (NO in Step S12), the correction processing is executed.

The observation control unit 791 of the correction control unit 79 acquires XYZ position information for the well 3 at the address designated in Step S11 from the storage unit 75 (Step S41). Subsequently, the observation control unit 791 selects the target grid 32T with which the tip end TE is brought into contact from the wells 3 at the address (Step S42). After the above, the Z correction value acquisition processing illustrated in Fig. 16 is executed (Step S43). By execution of Step S43 (Steps S1 to S7), first, a Z position correction value for the tip 10 currently attached to the head 61 is acquired.

After the above, the head control unit 72 raises the head 61 in a state where the tip end TE is in contact with the bottom surface 31 (Step S44). Due to the raising, the tip end TE is at a height position at which contact between the tip end TE and the bottom surface 31 and the grid 32 does not occur, and the tip end TE is clearly shown in an image of the bottom surface 31 captured by the camera unit 5. For example, the height position is 0.15 mm above the bottom surface 31.

After the head 61 is raised, the imaging control unit 73 causes the camera unit 5 to acquire an image of the bottom surface 31 near the target grid 32T (Step S45). The image processing unit 74 performs image processing such as contrast enhancement, edge detection, binarization processing, and noise removal processing on the acquired image data (Step S46). By the image processing, contours of the target grid 32T and the grid 32 around the target grid, and the tip end TE are image-recognized.

The correction value calculation unit 793 extracts XY coordinate positions of the target grid 32T and the tip end TE based on the image after the image processing (Step S47). Subsequently, the correction value calculation unit 793 calculates an XY movement correction value of the head 61 based on a difference between the XY coordinate position of the target grid 32T and the XY coordinate position of the tip end TE (Step S48). By the above processing, XYZ position correction values for the tip 10 currently attached to the head 61 are obtained for the well 3 at the address designated in Step S11. After the above, the processing proceeds to Step S14, and a picking operation of the cell C is executed.

The embodiment described above includes an invention shown below.

A cell handling apparatus according to one aspect of the present invention includes: a container including a translucent bottom surface that holds a cell, the bottom surface being deformable in shape and having a state checking portion for recognizing the shape deformation; a camera that images the bottom surface of the container from below; a head to which a tip having an end for sucking or discharging a cell is attached, the head being capable of executing an operation of approaching the bottom surface from above to suck a cell on the bottom surface and an operation of discharging a cell toward the bottom surface; and a correction control unit configured to correct position coordinates of the head, in which the correction control unit configured to acquire a correction value of the position coordinates of the head by obtaining a difference between a lowering target position of the tip end of the tip and an actual lowering position where the tip end of the tip actually comes into contact with the bottom surface, and detect contact of the tip end of the tip with the bottom surface based on a state change of the state checking portion in an image of the bottom surface captured by the camera.

Even when the head is lowered so as to direct the tip end of the tip to a lowering target position, a tip end position may be a position deviated from the lowering target position. According to the above aspect, a difference between a lowering target position and an actual lowering position where the tip end of the tip actually comes into contact with the bottom surface is obtained. That is, deviation between the actual movement position of the tip end and the lowering target position can be grasped with reference to the actual lowering position. Moreover, contact of the tip end with the container bottom surface is detected based on a state change of the state checking portion caused by the contact. Since the state change does not occur unless the tip end actually comes into contact with the bottom surface, the contact can be accurately determined. Therefore, accuracy of position control of the tip end is improved, and suction and discharge of a cell can be accurately performed as intended.

In the above-described cell handling apparatus, the state checking portion is desirably a deformation checking portion for checking a shape change of the bottom surface due to contact of the tip end of the tip with the bottom surface.

According to this aspect, since contact of the tip end with the bottom surface can be directly recognized as a shape change of the bottom surface, the contact can be more reliably grasped.

According to an aspect, the cell handling apparatus described above may be configured such that the bottom surface of the container is a bottom surface on which a large number of grids that contain a cell are regularly arranged, the deformation checking portion is the grid or an arrangement pattern of the grids, and the correction control unit detects contact of the tip end of the tip with the bottom surface based on a shape change of the grid or the arrangement pattern.

According to this aspect, the grid itself serving as a cell containing unit is utilized as a deformation checking portion for checking contact of the tip end with the container bottom surface. For this reason, it is possible to omit processing for separately providing a deformation checking portion on the bottom surface of the container.

In the cell handling apparatus described above, the correction control unit is desirably configured to select a target grid with which the tip end of the tip is brought into contact and a shape observation grid located around the target grid for observation of the shape change from among the large number of grids arranged on the bottom surface, and determine that the tip end of the tip comes into contact with the bottom surface when a shape change of the shape observation grid exceeds a predetermined threshold.

Rather than a grid actually in contact with the tip end of the tip, a grid around the above grid is used for easier checking of shape deformation on an image. According to the above aspect, since the latter grid is selected as the shape observation grid, it is possible to more accurately detect contact of the tip end with the bottom surface.

In the cell handling apparatus described above, the correction control unit is desirably configured to set one or a plurality of grids adjacent to the target grid as the shape observation grid.

In a grid adjacent to the target grid, shape deformation is likely to occur at an initial stage of contact of the tip end with the target grid. According to the above aspect, it is easy to accurately detect a timing at which the tip end comes into contact with the bottom surface.

In the cell handling apparatus described above, the correction control unit is desirably configured to observe an area of the shape observation grid in the image, and determines that the tip end of the tip is in contact with the bottom surface when the area is smaller than a predetermined threshold.

According to this aspect, contact of the tip end with the bottom surface is detected based on an area change of a grid. When a shape change occurs in a certain portion in an image observation region, the shape change is likely to appear as an area change of a grid in a two-dimensional image. Therefore, the shape change of the grid is easily detected by image processing.

In the cell handling apparatus described above, the correction control unit is desirably configured to observe a contour shape of the shape observation grid in the image, and determine that the tip end of the tip comes into contact with the bottom surface when a change rate of the contour shape is larger than a predetermined threshold.

According to this aspect, contact of the tip end with the bottom surface is detected based on a change rate of a contour shape of a grid. When the container bottom surface is deformed due to contact of the tip end, the deformation is likely to appear as a change in a contour shape of a grid. Therefore, the shape change of the grid is easily detected by image processing.

In the cell handling apparatus described above, the correction control unit is desirably further configured to perform control to correct positional misalignment in a horizontal direction of the tip end of the tip with respect to the target grid.

According to this aspect, positional misalignment can be corrected not only in the vertical direction in which the tip end of the tip is directed to the container bottom surface but also in the horizontal direction. Therefore, position control of the tip end can be more accurately performed.

In the cell handling apparatus described above, the correction control unit may be configured to lower the tip end of the tip to a predetermined position, cause the camera to image the bottom surface at every one pitch of the lowering, and observe a shape change of the shape observation grid.

According to this aspect, in a region where a shape change of the shape observation grid is likely to occur, the bottom surface is imaged at every one pitch of lowering, so that an occurrence timing of the shape change is easily detected. On the other hand, since imaging is not performed until the tip end reaches the predetermined position, takt time can be shortened.

According to the present invention, it is possible to provide a cell handling apparatus capable of accurately sucking and discharging a cell.

This application is based on Japanese Patent application No. 2025-20233 filed in Japan Patent Office on February 10, 2025, the contents of which are hereby incorporated by reference.

Although the present invention has been fully described by way of example with reference to the accompanying drawings, it is to be understood that various changes and modifications will be apparent to those skilled in the art. Therefore, unless otherwise such changes and modifications depart from the scope of the present invention hereinafter defined, they should be construed as being included therein.

## Claims

1. A cell handling apparatus (S) comprising:
a container (3) including a translucent bottom surface (31) that holds a cell (C), the bottom surface (31) being deformable in shape and having a state checking portion (32Q) for recognizing the shape deformation;
a camera (5) that images the bottom surface (31) of the container (3) from below;
a head (31) to which a tip (10) having a tip end (TE) for sucking or discharging a cell (C) is attached, the head (5) being capable of executing an operation of approaching the bottom surface (31) from above to suck a cell (C) on the bottom surface (31) and an operation of discharging a cell (C) toward the bottom surface (31); and
a correction control unit (79) configured to correct position coordinates of the head (61), wherein
the correction control unit (79) is configured to
acquire a correction value of the position coordinates of the head (61) by obtaining a difference between a lowering target position of the tip end (TE) of the tip (10) and an actual lowering position where the tip end (TE) of the tip (10) actually comes into contact with the bottom surface (31), and
detect contact of the tip end (TE) of the tip (10) with the bottom surface (31) based on a state change of the state checking portion (32Q) in an image of the bottom surface (31) captured by the camera (5).

2. The cell handling apparatus (S) according to claim 1, wherein the state checking portion (32Q) is a deformation checking portion for checking a shape change of the bottom surface (31) due to contact of the tip end (TE) of the tip (10) with the bottom surface (31).

3. The cell handling apparatus (S) according to claim 2, wherein
the bottom surface (31) of the container (3) is a bottom surface on which a large number of grids (32) that contain a cell (C) are regularly arranged,
the deformation checking portion (32Q) is the grid (32) or an arrangement pattern of the grids (32), and
the correction control unit (79) detects contact of the tip end (TE) of the tip (10) with the bottom surface (31) based on a shape change of the grid (32) or the arrangement pattern.

4. The cell handling apparatus (S) according to claim 3, wherein
the correction control unit (79) is configured to
select a target grid (32T) with which the tip end (TE) of the tip (10) is brought into contact and a shape observation grid (32Q) located around the target grid (32T) for observation of the shape change from among the large number of grids (32) arranged on the bottom surface (31), and
determine that the tip end (TE) of the tip (10) comes into contact with the bottom surface (31) when a shape change of the shape observation grid (32Q) exceeds a predetermined threshold.

5. The cell handling apparatus (S) according to claim 4, wherein the correction control unit (79) is configured to set one or a plurality of grids adjacent to the target grid (32T) as the shape observation grid (32Q).

6. The cell handling apparatus (S) according to claim 4, wherein the correction control unit (79) is configured to observe an area of the shape observation grid (32Q) in the image, and determines that the tip end (TE) of the tip (10) is in contact with the bottom surface (31) when the area is smaller than a predetermined threshold.

7. The cell handling apparatus (S) according to claim 4, wherein the correction control unit (79) is configured to observe a contour shape of the shape observation grid (32Q) in the image, and determine that the tip end (TE) of the tip (10) comes into contact with the bottom surface (31) when a change rate of the contour shape is larger than a predetermined threshold.

8. The cell handling apparatus (S) according to any one of claims 4 to 7, wherein the correction control unit (79) is further configured to perform control to correct positional misalignment in a horizontal direction of the tip end (TE) of the tip (10) with respect to the target grid (32T).

9. The cell handling apparatus (S) according to any one of claims 4 to 7, wherein the correction control unit (79) is configured to lower the tip end (TE) of the tip (10) to a predetermined position, cause the camera (5) to image the bottom surface (3 1) at every one pitch of the lowering, and observe a shape change of the shape observation grid (32Q).
